# FASCICULE DE BREVET EUROPEEN

(11) **EP 2 987 137 B1**
(45) Date de publication et mention de la délivrance du brevet: **25.11.2020**
(21) Numéro de dépôt: 14718969.0
(22) Date de dépôt: 18.04.2014
(51) Int. Cl.: G06T 7/11, G06T 7/136, G06T 7/149, A61N 5/10

(54) **PROCÉDÉ DE PRODUCTION DE DONNÉES REPRÉSENTATIVES DE CONTRAINTES DE TRAITEMENT DE RADIOTHÉRAPIE, DISPOSITIFS ET PROGRAMME CORRESPONDANT**
VERFAHREN ZUR HERSTELLUNG VON DATEN ZUR DARSTELLUNG VON RÖNTGENTHERAPIE-BEHANDLUNGSEINSCHRÄNKUNGEN, VORRICHTUNGEN UND ENTSPRECHENDES PROGRAMM
METHOD FOR PRODUCING DATA REPRESENTING RADIOTHERAPY TREATMENT CONSTRAINTS, DEVICES AND CORRESPONDING PROGRAM

(30) Priorité: 18.04.2013 FR 1353552
(43) Date de publication de la demande: 24.02.2016
(73) Titulaire: Université de Rennes I, 35065 Rennes Cedex (FR)
(72) Inventeur: ACOSTA, Oscar, F-35200 Rennes (FR); DREAN, Gaël, F-35000 Rennes (FR); DE CREVOISIER, Renaud, F-35700 Rennes (FR); HAIGRON, Pascal, F-35700 Rennes (FR)
(74) Mandataire: Vidon Brevets & Stratégie
(86) Numéro de dépôt international: PCT/EP2014/058025
(87) Numéro de publication internationale: WO 2014/170491

(56) Documents cités:
- WO-A1-2012/159671
- US-A1- 2012 320 055
- Sharif Qatarneh: "Development of a Whole Body Atlas for Radiation Therapy Planning and Treatment Optimization", , 31 décembre 2006 (2006-12-31), XP055091738, Extrait de l'Internet: URL:http://urn.kb.se/resolve?urn=urn:nbn:s e:su:diva-803 [extrait le 2013-12-05]
- WEN NING ET AL: "Evaluation of the deformation and corresponding dosimetric implications in prostate cancer treatment", PHYSICS IN MEDICINE AND BIOLOGY, INSTITUTE OF PHYSICS PUBLISHING, BRISTOL GB, vol. 57, no. 17, 7 septembre 2012 (2012-09-07), pages 5361-5379, XP002693995, ISSN: 0031-9155, DOI: 10.1088/0031-9155/57/17/5361 [extrait le 2012-08-03]
- RIZZO G ET AL: "Multi-modal medical image integration to optimize radiotherapy planning in lung cancer treatment", ANNALS OF BIOMEDICAL ENGINEERING, PERGAMON PRESS, OXFORD, GB, vol. 32, no. 10, 1 janvier 2004 (2004-01-01), pages 1399-1407, XP002488929, ISSN: 0090-6964, DOI: 10.1114/B:ABME.0000042227.37183.1C
- MIYABE YUKI ET AL: "New algorithm to simulate organ movement and deformation for four-dimensional dose calculation based on a three-dimensional CT and fluoroscopy of the thorax", MEDICAL PHYSICS, AIP, MELVILLE, NY, US, vol. 36, no. 10, 4 septembre 2009 (2009-09-04), pages 4328-4339, XP012129704, ISSN: 0094-2405, DOI: 10.1118/1.3213083

## Description

### 1. Domaine de l'invention

L'invention se rapporte au traitement de données d'imagerie médicale. L'invention se rapporte plus particulièrement à une méthode de traitement de données médicales en vue d'une planification, par une console de planification, d'une ou plusieurs actions à mener, comme par exemple des actions d'application d'un rayonnement sur une ou plusieurs zones données dans le cadre d'une radiothérapie.

On rappelle que la radiothérapie est une méthode de traitement de cancers qui utilise des radiations pour détruire les cellules cancéreuses. L'irradiation a pour but de détruire des cellules tumorales tout en épargnant des tissus sains périphériques. Pour permettre une irradiation, un accélérateur linéaire d'électron est utilisé, lequel produit un faisceau d'irradiation.

### 2. Art antérieur

L'histoire de la radiothérapie est relativement récente. La radiothérapie gagne en efficacité en fonction de l'évolution des progrès techniques. Ainsi, grâce à la mise en œ uvre d'un scanner, la planification des traitements de radiothérapie en trois-dimensions devient possible, ce qui représentait une avancée majeure par rapport aux traitements en deux dimensions. Les traitements basés sur l'utilisation du scanner permettent aux radiooncologues de déterminer plus précisément la distribution de la dose de radiation en utilisant des images tomodensitométriques de l'anatomie du patient. Des techniques d'imagerie ont permis l'apparition de la radiothérapie guidée par l'image (IGRT) qui permet de mieux contrôler la position de la zone à traiter au fur et à mesure du traitement. Des innovations au niveau des appareils de traitement comme l'apparition des collimateurs multi-lames ont permis l'apparition de la radiothérapie conformationnelle avec modulation d'intensité (RCMI) qui permet d'adapter plus précisément l'irradiation à la forme des organes à traiter. Il a été ainsi possible de visualiser et de traiter plus efficacement les tumeurs, ce qui s'est traduit par un meilleur pronostic pour les patients, une meilleure préservation des organes sains et moins d'effets secondaires.

Il subsiste cependant des problématiques. En effet, bien qu'il soit désormais possible de moduler l'intensité de l'irradiation en fonction de zones de traitement déterminées, il n'en reste pas moins que certaines régions anatomiques sont plus sensibles que d'autres aux irradiations. Ces zones de traitement peuvent être des organes, mais il s'agit également de portions d'organes. Il a été démontré que certaines portions d'organes, qui sont surirradiées, peuvent être responsables à elles-seules, d'une part conséquente des effets secondaires rencontrés lors du traitement par radiothérapie.

Ainsi, par exemple, lors d'un traitement d'un cancer des voies aéro-digestives supérieures (ou cancer ORL), il est particulièrement important de ne pas faire subir d'irradiation aux glandes salivaires. En effet, il a été démontré que l'irradiation de ces glandes génère des effets secondaires désastreux et contre-productifs comme par exemple la survenance d'un cancer des glandes salivaire elles-mêmes. Il est donc nécessaire de préserver ces glandes salivaires. Il en est de même pour le cancer de la prostate. Il a été démontré qu'une sous-région spécifique du rectum, lorsqu'elle est surirradiée, est corrélée à de possibles saignements rectaux qui surviennent postérieurement au traitement. Il est donc important de ne pas faire subir d'irradiation excessive à cette zone qui peut être plus sensible lors du traitement radiothérapique. D'autres zones et/ou d'autres organes sont également concernés.

Ces contraintes étant posées, il est nécessaire de connaître précisément la localisation de ces zones à éviter lors du traitement. Or ces zones ne sont la plupart du temps pas connues et dépendent des caractéristiques individuelles, de la forme du rectum, de son volume, de la forme de la vessie, du poids du patient, etc. Ainsi, à l'heure actuelle, il n'est pas possible de localiser, pour un patient spécifique, avec une précision suffisante, les sous-régions des organes qui doivent être évitées lors des traitements. En d'autres termes, des méthodes d'utilisation de l'information spatiale dans des modèles de prédiction, et leur inclusion dans une étape de planning en radiothérapie n'ont pas encore été rapportés. Il y a une énorme variabilité anatomique et clinique qui entrave le transfert des informations d'un modèle de population vers un patient spécifique.

En conclusion, il existe des matériels qui peuvent permettre d'éviter ces zones à risque et offrir une meilleure qualité de soins aux patients, mais il n'existe pas de méthodes permettant exploiter efficacement ces matériels.

Le document XP055091738 (Sharif Qatarneh: "Development of a Whole Body Atlas for Radiation Therapy Planning and Treatment Optimization", du 31 décembre 2006*),* concerne le développement d'un atlas dédié à la planification en Radiothérapie. Cet Atlas est basé sur une segmentation des organes entiers. La méthode décrite comprend notamment l'identification de régions à risque. Plus particulièrement, ces régions à risque se rapportent à **des organes ou des groupes d'organes entiers.** Les régions mises en évidence par la méthode de Qatarneh ne se rapportent pas à des sous-régions à risques au sein des organes étudiés. Par ailleurs, l'Atlas présenté par Qatarneh est établi à partir du modèle créé par le *« Visible Human project ».* Il est donc basé sur cette collection de données anatomiques qui ont étés obtenues grâce à l'étude de **deux** corps entiers. L'atlas formé par Qatarneh est issu de la segmentation des organes à partir de ces données relatives à deux corps entiers. Ainsi, l'utilisation de l'Atlas de Qatarneh permet d'accélérer la planification d'un traitement pour un patient donné, en permettant la segmentation des organes et des volumes de façon plus rapide. *Ce document ne propose pas cependant de baser une radio thérapie sur des régions à risques issues d'atlas combinés à une imagerie patient.*

Le document XP002693995 (WEN NING ET AL "Evaluation of the deformation and corresponding dosimetric implications in prostate cancer treatment", du 07 septembre 2012*.)* explique comment on peut comparer la dose planifiée à la dose réellement délivrée à un patient pendant le traitement, en utilisant les images prises au cours du traitement. Il se rapporte plus précisément à la mise en place de l'IGRT *(« Image-Guided Radiotherapy »). Ce document ne propose pas cependant de baser une radio thérapie sur des région à risques issues d'atlas.*

Le document WO 2012/159671 A1 (BRAINLAB AG [DE]; BERTRAM PASCAL [DE]; PROMBERGER CLAUS [DE]; FLURSCHU 29 novembre 2012). Ce document propose de réaliser des simulations de traitement radiothérapique. Il ne propose cependant pas de carte de probabilité d'effet secondaires basées sur un ou plusieurs atlas combinés à une imagerie du patient.

### 3. Résumé de l'invention

La technique proposée ne présente pas ces inconvénients de l'art antérieur. Plus particulièrement, la technique proposée se rapporte à un procédé de production de données représentatives de contraintes de traitement de radiothérapie associées à un patient à traiter. Un tel procédé est défini par la revendication 1.

Plusieurs modes de réalisation particuliers sont définis par les revendications dépendantes.

Dans un autre mode de réalisation, la technique se rapporte également à un dispositif de production de données représentatives de contraintes de traitement de radiothérapie associées à un patient à traiter. Un tel dispositif est défini par la revendication 7.

La technique décrite se rapporte également à un système de production de données représentatives de contraintes de traitement de radiothérapie associées à un patient à traiter. Un tel système est défini par la revendication 8.

Selon une implémentation préférée, les différentes étapes des procédés selon l'invention sont mises en œuvre par un ou plusieurs logiciels ou programmes d'ordinateur, comprenant des instructions logicielles destinées à être exécutées par un processeur de données d'un module relais selon l'invention et étant conçu pour commander l'exécution des différentes étapes des procédés.

En conséquence, l'invention vise aussi un programme, susceptible d'être exécuté par un ordinateur ou par un processeur de données, ce programme comportant des instructions pour commander l'exécution des étapes d'un procédé tel que mentionné ci-dessus.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un processeur de données, et comportant des instructions d'un programme tel que mentionné ci-dessus.

Le support d'informations peut être n'importe quelle entité ou dispositif capable de stocker le programme. Par exemple, le support peut comporter un moyen de stockage, tel qu'une ROM, par exemple un CD ROM ou une ROM de circuit microélectronique, ou encore un moyen d'enregistrement magnétique, par exemple une disquette (floppy disc) ou un disque dur.

D'autre part, le support d'informations peut être un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, le support d'informations peut être un circuit intégré dans lequel le programme est incorporé, le circuit étant adapté pour exécuter ou pour être utilisé dans l'exécution du procédé en question.

Selon un mode de réalisation, l'invention est mise en œuvre au moyen de composants logiciels et/ou matériels. Dans cette optique, le terme "module" peut correspondre dans ce document aussi bien à un composant logiciel, qu'à un composant matériel ou à un ensemble de composants matériels et logiciels.

Un composant logiciel correspond à un ou plusieurs programmes d'ordinateur, un ou plusieurs sous-programmes d'un programme, ou de manière plus générale à tout élément d'un programme ou d'un logiciel apte à mettre en œuvre une fonction ou un ensemble de fonctions, selon ce qui est décrit ci-dessous pour le module concerné. Un tel composant logiciel est exécuté par un processeur de données d'une entité physique (terminal, serveur, passerelle, routeur, etc.) et est susceptible d'accéder aux ressources matérielles de cette entité physique (mémoires, supports d'enregistrement, bus de communication, cartes électroniques d'entrées/sorties, interfaces utilisateur, etc.).

De la même manière, un composant matériel correspond à tout élément d'un ensemble matériel (ou hardware) apte à mettre en œuvre une fonction ou un ensemble de fonctions, selon ce qui est décrit ci-dessous pour le module concerné. Il peut s'agir d'un composant matériel programmable ou avec processeur intégré pour l'exécution de logiciel, par exemple un circuit intégré, une carte à puce, une carte à mémoire, une carte électronique pour l'exécution d'un micrologiciel (firmware), etc.

Chaque composante du système précédemment décrit met bien entendu en œuvre ses propres modules logiciels.

Les différents modes de réalisation mentionnés ci-dessus sont combinables entre eux pour la mise en œuvre de l'invention.

### 4. Figures

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :
- la figure 1 présente un synoptique de la technique proposée ;
- la figure 2 illustre la première phase de la technique proposée ;

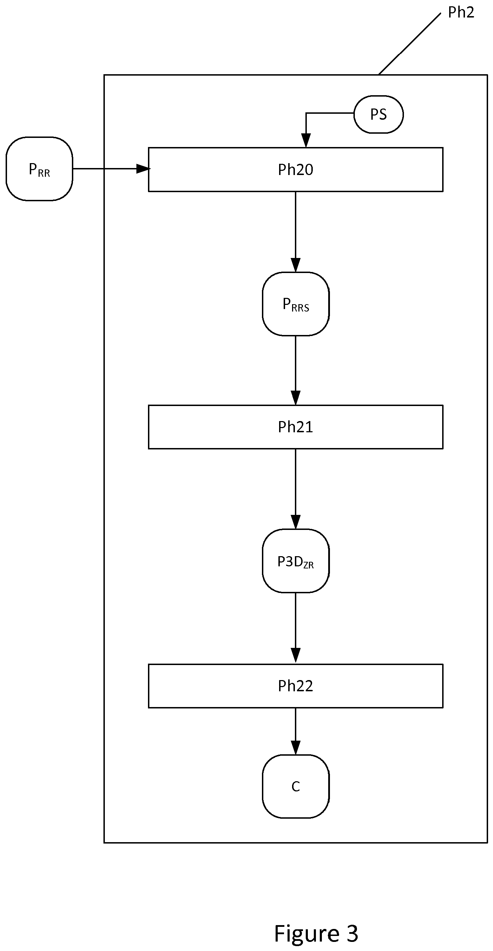

- la figure 3 illustre la deuxième phase de la technique proposée ;
- la figure 4 décrit un dispositif pour la mise en œuvre de la technique proposée.

### 5. Description d'un mode de réalisation

### 5.1. Rappel du principe de la technique

La technique proposée repose sur plusieurs éléments distincts. En premier lieu, la technique repose sur la création d'un ensemble de données appelé ensemble d'atlas. Un atlas est un ensemble de données représentatives de certaines caractéristiques. Plus particulièrement, un atlas comprend une image (une image médicale, de préférence en trois dimensions, comprenant des pixels en trois dimensions, nommés voxels) à laquelle plusieurs caractéristiques sont associées. Plus particulièrement, un atlas intègre les emplacements et les formes des structures anatomiques et les relations spatiales entre ces formes et ces structures.

En règle générale, on distingue deux catégories d'atlas. La première catégorie est directement obtenue à partir de données d'un seul patient. Il s'agit d'un atlas associé à un patient. La seconde catégorie est obtenue à partir de données correspondant à plusieurs patients. Dans ce cas, il s'agit d'atlas comprenant des données combinées, ou moyennes, dont l'objectif est de servir de référence. Un problème lié à ces atlas de l'art antérieur est qu'ils correspondent à un ensemble d'organes donnés. Plus particulièrement, dans le cas de données correspondant au cancer de la prostate, les atlas se rapportent à l'ensemble de la région prostatique, laquelle comprend outre la prostate, le rectum, la vessie, l'urètre, etc. Ces atlas ne se rapportent donc pas à un élément précis.

Comme l'objet de la technique décrite est d'éviter ou de diminuer les effets secondaires et que ces effets secondaires sont attribués à une irradiation, à une trop forte dose, d'une ou plusieurs zones sensibles à l'intérieur des organes et que ces zones sensibles ne sont pas a priori connues pour un patient donné, il est nécessaire d'utiliser un autre type d'atlas.

Ce nouveau type d'atlas est de type combinatoire, comprenant une image en trois dimensions issue de la combinaison de plusieurs images en trois dimensions d'origine. Ces images en trois dimensions d'origine sont soit issues d'un même patient (dans ce cas les images d'origine ont été obtenues à des moments différents), soit issues de plusieurs patients. On dispose donc, dans cet atlas, de données anatomiques combinées. Ce nouveau type d'atlas présente la particularité de comprendre, en plus des données anatomiques combinées, des données cliniques obtenues postérieurement. Parmi les données cliniques obtenues postérieurement, on a notamment la survenance d'effets secondaires. Dans le cas du cancer de la prostate, il s'agit par exemple de saignements du rectum. Dans le cas d'un cancer ORL, il s'agit par exemple de la perte du gout ou de l'absence de salive. Dans ce nouvel atlas, on dispose également d'une distribution de doses combinée. Cette distribution de dose de l'atlas résulte d'un calcul réalisé sur des distributions de doses unitaires. Ces distributions de doses unitaires sont celles qui sont associées aux images unitaires qui ont été utilisées pour produire l'image combinée comprise dans l'atlas. En fonction des données cliniques (effets secondaires), des distributions de doses, on produit un atlas à partir duquel une analyse par voxel peut être mise en œuvre pour déterminer la dose reçue pour un ou un ensemble de voxels donné et corréler cette information à la survenance des effets secondaires. Un atlas, dans la technique proposée, porte donc une information associée à une zone probable liée aux effets secondaires mesurés. Ainsi, à partir de données de plusieurs patients dans une population, une méthode automatique permet de construire au moins un atlas qui définit des sous-régions à l'intérieur des organes qui sont en relation avec la dose et la toxicité.

L'idée, à la base de la technique proposée, est de disposer de plusieurs atlas de ce type. Ces atlas sont regroupés dans un ensemble d'atlas. Les atlas de cet ensemble d'atlas sont disjoints par au moins une caractéristique (au moins une donnée clinique et/ou au moins une donnée anatomique).

Compte tenu de la disponibilité des effets secondaires associés aux patients, on dispose, pour un atlas courant de l'ensemble d'atlas, en fonction de la distribution de dose combinée, d'une localisation probable d'une zone ayant contribuée à la survenance de l'effet secondaire que l'on souhaite éviter (il s'agit de données probabilistes de localisation de zones associées audit atlas courant). Ce qui vient d'être exposé constitue le principe de la technique proposée.

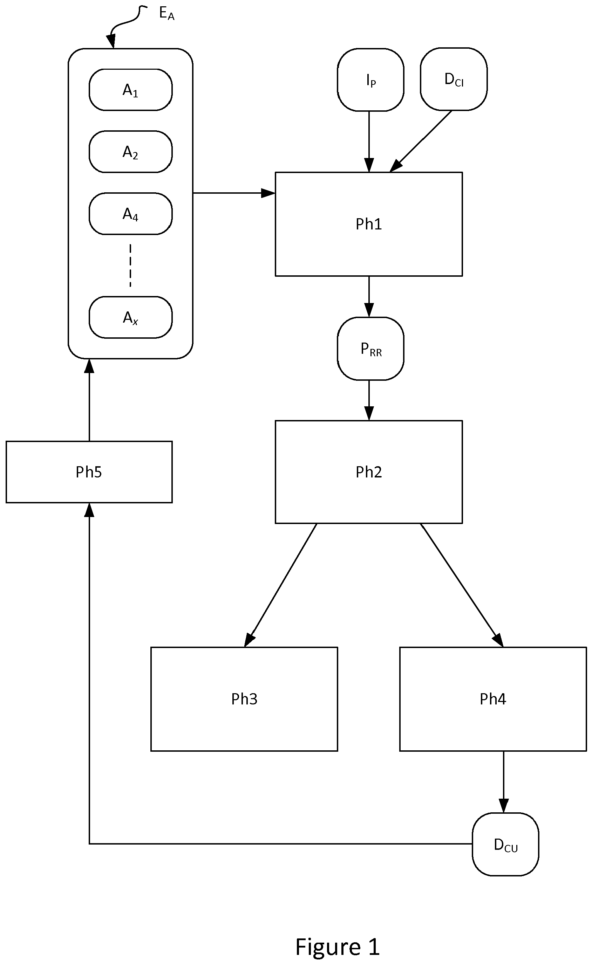

On décrit, en relation avec la figure 1, les différentes phases de la technique proposée. En premier lieu, on dispose d'un ensemble d'atlas (E_{(A)}), comprenant x atlas (A₁ à Aₓ). Cet ensemble d'atlas est utilisé, dans une première phase (Ph1), pour être mis en correspondance avec un patient à planifier pour lequel on dispose d'une part d'une image (I_{P}) et d'autre part de données cliniques initiales (D_{CI}). Ces données cliniques initiales se rapportent par exemple à l'age, au poids, à la taille, aux antécédents médicaux et à la pathologie cancéreuse.

Ainsi, à l'aide de l'ensemble d'atlas (E_{(A)}) et d'au moins une image en trois dimensions (I_{P}) correspondant à un patient à planifier (et à traiter), on réalise au moins une mise en correspondance des atlas de l'ensemble des atlas et du patient à planifier. Ainsi, pour un nouveau patient à planifier, le(s) atlas sont mis en correspondance (spatiale, clinique, temporelle). De manière complémentaire, lorsque plusieurs atlas sont successivement mis en correspondance avec l'image de base, ces atlas peuvent être pondérés en tenant compte de la similarité entre chaque atlas et le patient lors de la mise en correspondance.

Cette phase de mise en correspondance permet d'obtenir, pour le patient à planifier, une carte de probabilité de région à risque (P_{RR}). Cette carte de probabilité permet de situer, sur le patient à planifier, la zone ou les zones qui seront, pour ce patient, les plus probablement à l'origine d'effets secondaires. Cette carte est un ensemble de données comprenant, pour un voxel donné, une probabilité de se trouver dans une région associée à une radio-sensibilité plus importante. La deuxième phase (Ph2) consiste à générer des contraintes spécifiques au patient, en fonction de la carte de probabilité de région à risque (P_{RR}). Ces contraintes spécifiques au patient peuvent être générées par la console de traitement, de manière automatique. On appelle contrainte (C) une structure de données représentative d'une zone ou sous région que l'on suppose être sensible aux radiations. Cette structure de données peut avoir plusieurs formes. Une première forme est une image tridimensionnelle binaire. Cette image comprend des 0 aux voxels qui ne font pas partie de la zone à risque et des 1 aux voxels qui font partie de la zone à risque. Une deuxième forme est une image tridimensionnelle probabiliste dans laquelle les voxel portent une information probabiliste. Une autre forme est une structure de données descriptive, identifiant les coordonnées de la zone à risque dans l'espace de coordonnées de la console de planification.

Les zones à l'intérieur des organes à risques étant identifiées, la troisième phase (Ph3) consiste à effectuer un planning de traitement du patient. Cette tâche est également mise en œuvre de manière automatique par l'intermédiaire de la console de traitement. Les contraintes permettent à la console de planifier le traitement de ce patient par exemple en évitant les sous-régions des organes à risques ou en réduisant la dose planifié sur ces mêmes sous-régions. En fonction de la forme que prennent les contraintes, l'intégration de celles-ci dans la console de planification est modifiée.

La quatrième phase (Ph4), qui se tient en parallèle de la troisième, consiste à mettre en œuvre une prédiction de la toxicité pour le patient en fonction des caractéristiques individuelles et des nouvelles contraintes générées au regard d'un traitement en IMRT, ARC, Cyber-Knife, qui permettent le ciblage de petites régions de manière précise. Cette planification a pour but de diminuer le risque de toxicité lié à la zone délimité. Plus particulièrement, cette phase de prédiction comprend au moins une étape d'évaluation d'une dose reçue par ledit patient lors dudit traitement, comme par exemple au moins une étape de calcul d'une dose volumique reçue au sein de ladite zone à risque en fonction de la carte de probabilité de région à risque (P_{RR}) et au moins une étape de calcul d'une probabilité de toxicité résultante de l'application d'une dose sur ladite zone à risque. Ce calcul est itéré autant de fois qu'il y a de séances de traitement afin d'obtenir une probabilité de survenance d'effet secondaire du traitement. Dans un contexte de contrôle de la tumeur et de diminution de la toxicité, la technique proposée fournit donc un ensemble d'étapes nécessaires à la personnalisation du traitement avec une quantification possible du bénéfice en termes de toxicité du traitement.

Pour finir, la dernière phase (Ph5) consiste à réintroduire, sous une forme anonyme, dans l'ensemble d'atlas, les données cliniques ultérieures (D_{CU}) à l'issue de la troisième phase dans l'ensemble d'atlas (E_{(A)}). Plus particulièrement, à partir de l'introduction de ces nouvelles données, il est possible de recalculer un nouvel ensemble d'atlas, lequel prend en considération notamment les effets secondaires du patient qui vient d'être traité.

### 5.2. Description d'un mode de réalisation

Dans ce mode de réalisation, la technique précédemment décrite est appliquée au traitement du cancer de la prostate. Dans un contexte d'individualisation du traitement de radiothérapie par modulation d'intensité (IMRT), la technique présentée dans ce mode de réalisation génère des contraintes spatiales spécifiques à un patient donné dans le but de réduire la toxicité des doses et/ou de prédire la toxicité des doses pour des organes ou des zones à risque. Plus particulièrement, un objet de cette technique, dans une application particulière est de réduire les saignements rectaux, voire de supprimer leur apparition.

On considère, dans ce mode de réalisation, que l'on dispose d'un ensemble d'atlas (E_{(A)}). Cet ensemble d'atlas est construit par une méthode de construction d'atlas qui est décrite par la suite. Cet ensemble d'atlas est construit préalablement à l'application de la présente technique. Selon une première variante, il est construit immédiatement avant la mise en œuvre de la présente technique. Selon une deuxième variante, l'ensemble d'atlas est construit en amont, par exemple lors d'une phase d'installation ou de paramétrage d'un dispositif de traitement (une console de traitement). Selon une troisième variante l'atlas est disponible par l'intermédiaire d'un réseau de communication et est par exemple mutualisé entre différents équipements (différentes consoles) et la technique proposée est mise en œuvre sur ce réseau de communication.

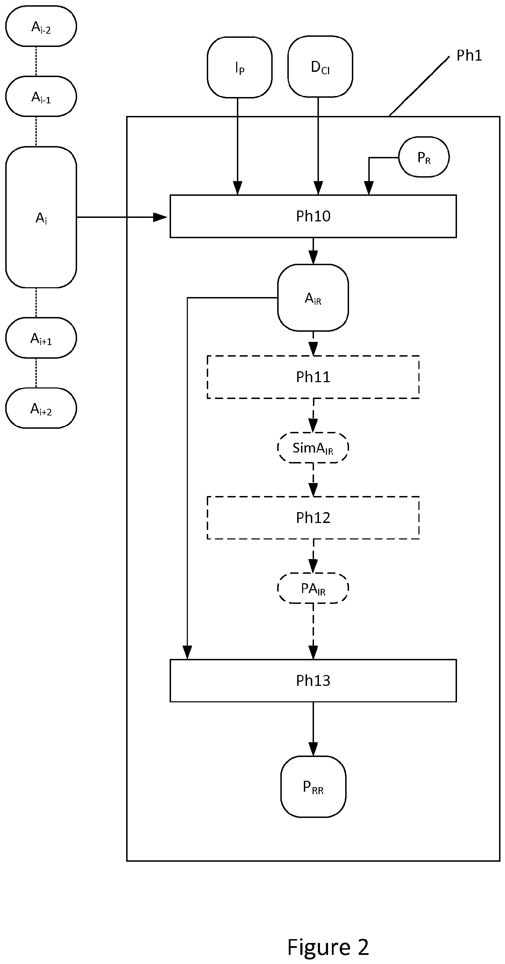

Dans ce mode de réalisation la technique comprend :
- une phase (Ph1) de mise en correspondance d'au moins un atlas courant (Aᵢ) dudit ensemble d'atlas (E_{(A)}) en fonction d'au moins une donnée représentative d'un patient à traiter, délivrant la carte de probabilité de région à risque (P_{RR}) ;
- une phase de génération (Ph2) d'au moins une donnée représentative d'une contrainte (C) à appliquer à un traitement destiné audit patient en fonction de ladite carte de probabilité de région à risque (P_{RR}).
   Plus particulièrement, en relation avec la figure 2, la phase de mise en correspondance (Ph1) comprend, pour ledit atlas courant (Aᵢ) dudit ensemble d'atlas (E_{(A)}) :
- une étape de recalage (Ph10), à partir d'au moins un paramètre de recalage déterminé (P_{R}), d'au moins une partie desdites données dudit atlas courant (Aᵢ) sur ladite au moins une donnée représentative dudit patient (I_{P}, D_{Cl}), délivrant un atlas courant recalé (A_{IR}) contenant des données probabilistes brutes ;
Cette phase (Ph1) de mise en correspondance peut être mise en œuvre pour chaque atlas dudit ensemble d'atlas (E_{(A)}).

En fonction d'un paramètre de configuration, cette phase comprend en outre :
- une étape de calcul d'une similarité (Ph11) entre ledit atlas courant recalé (A_{IR}) et ladite au moins une donnée représentative dudit patient (I_{P}, D_{Cl}), délivrant une valeur de similarité (SimA_{IR}) ; et
- une étape de pondération (Ph12) de données probabilistes contenues dans ledit atlas courant recalé (A_{IR}) en fonction de ladite valeur de similarité délivrant une valeur de pondération (PA_{IR}) ;

Par ailleurs, la phase de mise en correspondance comprend également :
- une étape de calcul (Phl3), à l'aide de ladite au moins une donnée représentative dudit patient (I_{P}, D_{CI}), de ladite carte de probabilité de région à risque (P_{RR}) en fonction soit desdites données probabilistes brutes, soit en fonction des données probabilistes pondérées.

Lorsque l'ensemble d'atlas comprend plusieurs atlas, l'étape de calcul Ph13 est réalisée à l'aide des données des atlas qui ont fait l'objet d'un recalage. Ainsi, les étapes Ph10, Ph11 et Ph12 peuvent être répétées autant de fois qu'il y a d'atlas dans l'ensemble d'atlas (E_{(A)}) ou sur un sous ensemble des atlas de l'ensemble d'atlas (E_{(A)}). Ceci est représenté sur la figure par les Atlas Aₗ₋₂ à Aₗ₊₂. Dès lors, l'étape Ph13 est soit mise en œuvre au coup par coup en fonction des résultats des étapes précédentes, soit directement en compilant en une seule étape les recalages et les pondérations précédemment réalisées.

On dispose ainsi, à l'issue de cette phase, de données probabilistes (P_{RR}), en quelque sorte calquées sur les données du patient, et plus particulièrement sur les données anatomiques du patient, sous la forme d'une carte de probabilité de région à risque (P_{RR}) en trois dimensions. Cette carte comprend, pour chaque voxel, en sus des données usuelles, une probabilité d'une présence d'une zone à risque. Les précédentes étapes sont mises en œuvre de manière automatique, soit par la console de traitement elle-même, soit par l'intermédiaire d'une ressource de calcul accessible par la voie d'un réseau de communication.

Plus particulièrement, la phase de génération (Ph2) de ladite au moins une donnée représentative d'une contrainte (C) comprend :
- une étape de seuillage (Ph20) de la carte de probabilité de région à risque (P_{RR}) en fonction d'un paramètre de seuillage prédéterminé (PS). Dans un mode de réalisation spécifique, ledit paramètre de seuillage est égal à 0,5. On considère ainsi que toute probabilité d'une sensibilité inférieure à 0,5 équivaut à une absence de sensibilité et qu'une probabilité d'une sensibilité supérieure ou égale à 0,5 équivaut à une sensibilité.
   Dans un mode de réalisation, cette étape de seuillage est mise en œuvre sur la carte de probabilité de région à risque (P_{RR}) indépendamment voxel par voxel. Dans un autre mode de réalisation, cette étape de seuillage est mise en œuvre de manière ensembliste, en considérant par exemple qu'un voxel de faible probabilité (inférieure à 0,5) qui comprend dans son voisinage un certain nombre de voxel (par exemple plus de treize) de forte probabilité (supérieure à 0,5) est un anachronisme et est considéré comme un voxel à forte probabilité. En d'autres termes, le procédé comprend une étape de seuillage des probabilités de présence d'une zone sensible en fonction d'un paramètre de seuillage prédéterminé, délivrant au moins une donnée représentative d'une présence d'une zone sensible.
- une étape d'obtention (Ph21) en fonction de ladite carte de probabilité de région à risque seuillée (P_{RRS}), d'au moins une position tridimensionnelle de ladite zone à risque pour ledit patient (P3D_{ZR}). L'obtention de cette position peut soit être réalisée par la transmission, par l'intermédiaire d'un réseau de communication, d'une position calculée au sein du réseau ou encore par l'intermédiaire d'un calcul réalisé au sein de la console de traitement elle-même. Dans au moins un mode de réalisation particulier, la zone à risque est assimilée à un organe au sein de ladite console. Ceci permet de contourner une impossibilité, pour certaines consoles, d'identifier des zones particulière de certains organes. En d'autres termes, on a une étape d'intégration, au sein d'une application de planification de traitement, des données représentatives d'une présence d'une zone sensible.
- une étape de génération (Ph22) ladite au moins une donnée représentative d'une contrainte (C) de traitement en fonction de ladite position (P3D_{ZR}).

On dispose ainsi, à l'issue de cette phase, de contraintes associées au patient dans le cadre du traitement qui lui est destiné. Ces contraintes peuvent soit prendre la forme de doses spécifiques à appliquer à la ou les zones à risques identifiées. Ces contraintes peuvent également se traduire par un ensemble de coordonnées spatiales définies dans la console et marquées de manière spécifiques pour l'opérateur de traitement de radiothérapie afin qu'il détermine lui-même les doses qui vont être appliquées.

### 5.3. Construction de l'ensemble d'atlas

L'ensemble d'atlas (E_{(A)}) est un modèle multi-varié de prédiction de toxicité. C'est un résultat d'une analyse locale (par voxel) de doses individuelles sur une population donnée. En d'autres termes, à la différence des atlas usuels, l'ensemble d'atlas (E_{(A)}) de la présente technique comprend des données cliniques corrélées aux doses planifiées ou effectivement reçues par les patients. Ces données sont intégrées à l'atlas. Ainsi, cet ensemble d'atlas est un modèle multi-varié (comprenant plusieurs grades de toxicité, plusieurs espaces de référence, plusieurs types de variables cliniques) résultat d'une analyse de population. Il permet d'établir des relations entre des distributions de doses en trois dimensions, obtenues lors de la planification ou par mesure et la toxicité de ces doses reçues. Plusieurs espaces de référence (ou atlas) sont utilisés en fonction de critères de similarité inter-individus.

On connaît, dans les techniques antérieures, des méthodes permettant d'obtenir un atlas à partir d'une population donnée. À la différence de ces techniques antérieure, la technique décrite ici permet d'obtenir plusieurs atlas à partir d'une population donnée. Ces différents atlas peuvent être obtenus à partir d'une méthode de base en revanche, les différents atlas correspondent chacun à un paramètre préalablement déterminé. En effet, dans les techniques antérieures, un atlas correspond à un ensemble de données correspondant à une synthèse de paramètres. Dans la technique objet de la présente demande, un atlas correspond à une synthèse orientée à partir d'un seul paramètre de départ.

Plus précisément, la technique ici présentée comprend :
- une phase de reconnaissance et de marquage, par un ou plusieurs méthodes experts, de la position d'un ou plusieurs sous-régions des organes dans les différentes images destinées à l'atlas ; Les experts utilisés sont des méthodes automatique (basées sur des analyses de population).
- une phase de détermination, parmi les images précédentes, d'une image de référence ;
- une phase d'application, en fonction d'une pluralité de doses préalablement connues, d'une distribution de doses sur les images marquées. Cette phase d'application peut être mise en œuvre par l'intermédiaire d'une déformation appliquée sur les images en fonction d'une image de référence ;
- une phase de normalisation des images en fonction de l'image de référence ;
- une phase de détermination d'une distribution de dose normalisée à l'aide des informations précédentes.

Ainsi, on dispose d'une distribution de dose cohérente, appliquée sur un patient particulier.

### 5.4. Dispositif pour la mise en œuvre de la technique proposée

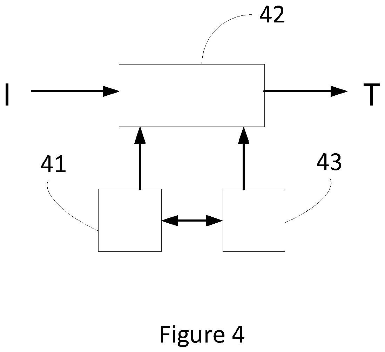

On présente, en relation avec la figure 4, une architecture simplifiée d'un dispositif apte à mettre en œuvre la technique décrite. Un tel dispositif comprend une mémoire 41, une unité de traitement 42 équipée par exemple d'un microprocesseur, et pilotée par le programme d'ordinateur 43, mettant en œuvre au moins une partie du procédé tel que décrit. Dans au moins un mode de réalisation, l'invention est mise en œ uvre sous la forme d'une application installée sur un dispositif de planification. Un tel dispositif comprend :
- des moyens de mise en correspondance d'au moins un atlas courant (Aᵢ) dudit ensemble d'atlas (E_{(A)}) en fonction d'au moins une donnée représentative d'un patient à traiter, délivrant la carte de probabilité de région à risque (P_{RR}). Ces moyens peuvent se matérialiser sous la forme d'une interface de connexion (I) à un ou plusieurs réseaux de communication. Il peut s'agir d'interfaces logicielles ou d'interfaces matérielles (de type carte réseau ou modules matériels de communication réseau). Ces moyens peuvent se présenter sous la forme de ressources de calcul dédiées permettant de réaliser les traitements au sein même du dispositif;
- des moyens de génération (Ph2) d'au moins une donnée représentative d'une contrainte (C) à appliquer à un traitement destiné audit patient en fonction de ladite carte de probabilité de région à risque (P_{RR})
- des moyens de transmission (T), à un dispositif de traitement, des données précédemment générées.

Dans au moins un mode de réalisation, la technique décrite peut être mis en œuvre par l'intermédiaire d'un réseau de communication auquel un dispositif est connecté. Dans au moins un mode de réalisation, la technique décrite est mise en œuvre au sein d'une console de planification de traitement de radiothérapie.

## Revendications

1. Procédé de production de données représentatives de contraintes de traitement de radiothérapie associées à un patient à traiter, procédé **caractérisé en ce qu'**il comprend :
- une phase (Ph1) de mise en correspondance d'au moins un atlas courant (Aᵢ) d'un ensemble d'atlas (E_{(A)}) avec au moins une donnée représentative comprenant au moins une image représentative d'un patient à traiter, ledit atlas contenant des sous-régions d'organes à risque, délivrant une carte de probabilité de sous-régions à risque (P_{RR}), ladite carte de probabilité étant un ensemble de données comprenant, pour un voxel donné, une probabilité de se trouver dans une région associée à une radiosensibilité plus importante ;
- une phase de génération (Ph2) d'au moins une donnée représentative d'une contrainte (C) à appliquer à un traitement destiné audit patient en fonction de ladite carte de probabilité de sous-régions à risque (P_{RR}).

2. Procédé selon la revendication 1, **caractérisé en ce qu'**il comprend en outre une phase de planification d'au moins un traitement de radiothérapie en fonction de ladite au moins une donnée représentative d'une contrainte (C).

3. Procédé selon la revendication 1, **caractérisé en ce qu'**il comprend en outre une phase de prédiction d'une toxicité pour ledit patient en fonction de ladite au moins une image représentative dudit patient à traiter de ladite au moins une donnée représentative d'une contrainte (C) générée.

4. Procédé selon la revendication 1, **caractérisé en ce que** ladite phase (Ph1) de mise en correspondance d'au moins un atlas courant (Aᵢ) dudit ensemble d'atlas (E_{(A)}) comprend :
- une étape de recalage, à partir d'au moins un paramètre de recalage déterminé, d'au moins une partie desdites données dudit atlas courant sur ladite au moins une image représentative dudit patient, délivrant un atlas courant recalé contenant des données probabilistes brutes ;
- une étape de calcul, à l'aide de ladite au moins une image représentative dudit patient, de ladite carte de probabilité de sous-régions à risque (P_{RR}) en fonction desdites données probabilistes brutes.

5. Procédé selon la revendication 4, **caractérisé en ce que** ladite phase (Ph1) de mise en correspondance d'au moins un atlas courant (Aᵢ) dudit ensemble d'atlas (E_{(A)}) comprend en outre :
- une étape de calcul d'une similarité entre ledit atlas courant et ladite au moins une image représentative dudit patient, délivrant une valeur de similarité ; et
- une étape de pondération de données probabilistes contenues dans ledit atlas courant recalé en fonction de ladite valeur de similarité ;
- et **en ce que** ladite une étape de calcul, à l'aide de ladite au moins une image représentative dudit patient, de ladite carte de probabilité de région à risque (P_{RR}) est réalisée en fonction des données probabilistes pondérées.

6. Procédé selon la revendication 1, **caractérisé en ce que** une ladite phase de génération (Ph2) d'au moins une donnée représentative d'une contrainte (C) comprend :
- une étape de seuillage (Ph20) de la carte de probabilité de sous-régions à risque (P_{RR}) en fonction d'un paramètre de seuillage prédéterminé (PS) ;
- une étape d'obtention en fonction de ladite carte de probabilité de sous-régions à risque seuillée, d'au moins une position tridimensionnelle de ladite zone à risque pour ledit patient.
- une étape de génération ladite au moins une donnée représentative d'une contrainte (C) de traitement en fonction de ladite position.

7. Dispositif de production de données représentatives de contraintes de traitement de radiothérapie associées à un patient à traiter, dispositif **caractérisé en ce qu'**il comprend :
- des moyens de mise en correspondance d'au moins un atlas courant (Aᵢ) d'un ensemble d'atlas (E_{(A)}) avec au moins une donnée représentative comprenant au moins une image représentative d'un patient à traiter, ledit atlas contenant des sous-régions d'organes à risque, délivrant une carte de probabilité de sous-régions à risque (P_{RR}), ladite carte de probabilité étant un ensemble de données comprenant, pour un voxel donné, une probabilité de se trouver dans une région associée à une radiosensibilité plus importante ;
- des moyens de génération (Ph2) d'au moins une donnée représentative d'une contrainte (C) à appliquer à un traitement destiné audit patient en fonction de ladite carte de probabilité de sous- région à risque (P_{RR}).

8. Système de production de données représentatives de contraintes de traitement de radiothérapie associées à un patient à traiter, système **caractérisé en ce qu'**il comprend :
- un premier dispositif qui comprend des moyens de mise en correspondance d'au moins un atlas courant (Ai) d'un ensemble d'atlas (E(A)) avec au moins une donnée représentative comprenant au moins une image représentative d'un patient à traiter, ledit atlas contenant des sous-régions d'organes à risque, délivrant une carte de probabilité de sous-régions à risque (PRR), ladite carte de probabilité étant un ensemble de données comprenant, pour un voxel donné, une probabilité de se trouver dans une région associée à une radiosensibilité plus importante ;
- un deuxième dispositif qui comprend des moyens de génération (Ph2) d'au moins une donnée représentative d'une contrainte (C) à appliquer à un traitement destiné audit patient en fonction de ladite carte de probabilité de sous-régions à risque (PRR).

9. Système de production de données selon la revendication 8, **caractérisé en ce qu'**il comprend des moyens de prédiction d'une toxicité pour ledit patient en fonction de ladite au moins une image représentative dudit patient à traiter et de ladite au moins une donnée représentative d'une contrainte (C) générée.

10. Produit programme d'ordinateur téléchargeable depuis un réseau de communication et/ou stocké sur un support lisible par ordinateur et/ou exécutable par un microprocesseur, **caractérisé en ce qu'**il comprend des instructions de code de programme pour l'exécution d'un procédé de fourniture selon la revendication 1, lorsqu'il est exécuté sur un ordinateur.

## Patentansprüche

1. Verfahren zur Generierung von einem zu behandelnden Patienten zugeordneten Daten, die für Behandlungseinschränkungen in der Strahlentherapie repräsentativ sind, Verfahren **dadurch gekennzeichnet, dass** es aufweist:
- eine Phase (Ph1) des Abgleichens mindestens eines aktuellen Atlasses (Aᵢ) eines Satzes von Atlanten (E_{(A)}) mit mindestens einer repräsentativen Dateneinheit, die mindestens ein für einen zu behandelnden Patienten repräsentatives Bild aufweist, wobei der Unterregionen gefährdeter Organe enthaltende Atlas eine Wahrscheinlichkeitskarte der gefährdeten Unterregionen (P_{RR}) bereitstellt, wobei die Wahrscheinlichkeitskarte ein Datensatz ist, der für ein gegebenes Voxel eine Wahrscheinlichkeit aufweist, sich in einer Region zu befinden, der eine höhere Strahlenempfindlichkeit zugeordnet ist,
- eine Phase des Generierens (Ph2) mindestens einer für eine Einschränkung (C) repräsentativen Dateneinheit zur Anwendung auf eine für den Patienten bestimmte Behandlung als Funktion der Wahrscheinlichkeitskarte der gefährdeten Unterregionen (P_{RR}).

2. Verfahren nach Anspruch 1, **dadurch gekennzeichnet, dass** es ferner eine Phase des Planens mindestens einer Strahlentherapiebehandlung als Funktion der für eine Einschränkung (C) repräsentativen Dateneinheit aufweist.

3. Verfahren nach Anspruch 1, **dadurch gekennzeichnet, dass** es ferner eine Phase des Vorhersagens einer Toxizität für den Patienten als Funktion des mindestens einen für den zu behandelnden Patienten repräsentativen Bilds der für eine Einschränkung (C) repräsentativen mindestens einen generierten Dateneinheit aufweist.

4. Verfahren nach Anspruch 1, **dadurch gekennzeichnet, dass** die Phase (Ph1) des Abgleichens mindestens eines aktuellen Atlasses (Aᵢ) aus dem Satz von Atlanten (E_{(A)}) aufweist:
- einen Schritt der Neueinstellung, ausgehend von mindestens einem bestimmten Neueinstellungsparameter, mindestens eines Teils der Daten des aktuellen Atlasses auf dem mindestens einen für den Patienten repräsentativen Bilds, der einen aktuellen neu eingestellten Atlas bereitstellt, welcher probabilistische Rohdaten enthält,
- einen Schritt des Berechnens, mit Hilfe des mindestens einen für den Patienten repräsentativen Bilds, der Wahrscheinlichkeitskarte der gefährdeten Unterregionen (P_{RR}) als Funktion der probabilistischen Rohdaten.

5. Verfahren nach Anspruch 4, **dadurch gekennzeichnet, dass** die Phase (Ph1) des Abgleichens mindestens eines aktuellen Atlasses (Aᵢ) aus dem Satz von Atlanten (E_{(A)}) ferner aufweist:
- einen Schritt des Berechnens einer Ähnlichkeit zwischen dem aktuellen Atlas und dem mindestens einen für den Patienten repräsentativen Bild, der einen Ähnlichkeitswert bereitstellt, und
- einen Schritt des Bewertens der probabilistischen Daten, die in dem neu eingestellten Atlas enthalten sind, als Funktion des Ähnlichkeitswerts,
- und dadurch, dass der Schritt des Berechnens, mit Hilfe des mindestens einen für den Patienten repräsentativen Bilds, der Wahrscheinlichkeitskarte der gefährdeten Unterregionen (P_{RR}) als Funktion der probabilistischen Rohdaten durchgeführt wird.

6. Verfahren nach Anspruch 1, **dadurch gekennzeichnet, dass** die Phase des Generierens (Ph2) von mindestens einer für eine Einschränkung (C) repräsentativen Dateneinheit, aufweist:
- einen Schritt des Schwellenwertvergleichens (Ph20) der Wahrscheinlichkeitskarte der gefährdeten Unterregionen (P_{RR}) als Funktion eines vorbestimmten Schwellenwertvergleichsparameters (PS),
- einen Schritt des Erhaltens, als Funktion der Wahrscheinlichkeitskarte der gefährdeten Unterregionen, die einem Schwellenwertvergleich unterzogen wurde, mindestens einer dreidimensionalen Position des Gefahrenbereichs für den Patienten,
- einen Schritt des Generierens der mindestens einen für eine Einschränkung (C) der Behandlung repräsentativen Dateneinheit als Funktion der Position.

7. Vorrichtung zur Generierung von für Einschränkungen der Strahlentherapiebehandlung repräsentativen Daten, die einem zu behandelnden Patienten zugeordnet sind, Vorrichtung **dadurch gekennzeichnet, dass** sie aufweist:
- Mittel zum Abgleichen mindestens eines aktuellen Atlasses (Aᵢ) eines Satzes von Atlanten (E_{(A)}) mit mindestens einer repräsentativen Dateneinheit, die mindestens ein für einen zu behandelnden Patienten repräsentatives Bild aufweist, wobei der Atlas, der Unterregionen gefährdeter Organe enthält, eine Wahrscheinlichkeitskarte der gefährdeten Unterregionen (P_{RR}) bereitstellt, wobei die Wahrscheinlichkeitskarte ein Datensatz ist, der für ein gegebenes Voxel eine Wahrscheinlichkeit aufweist, sich in einer Region zu befinden, die mit einer höheren Strahlenempfindlichkeit verbunden ist,
- Mittel zum Generieren (Ph2) mindestens einer für eine Einschränkung (C) repräsentativen Dateneinheit zur Anwendung auf eine für den Patienten bestimmte Behandlung als Funktion der Wahrscheinlichkeitskarte der gefährdeten Unterregionen (P_{RR}).

8. System zum Generieren von für Einschränkungen der Strahlentherapiebehandlung repräsentativen Daten, die einem zu behandelnden Patienten zugeordnet sind, System **dadurch gekennzeichnet, dass** es aufweist:
- eine erste Vorrichtung, die Mittel zum Abgleichen mindestens eines aktuellen Atlasses (Aᵢ) eines Satzes von Atlanten (E_{(A)}) mit mindestens einer repräsentativen Dateneinheit aufweist, die mindestens ein für einen zu behandelnden Patienten repräsentatives Bild aufweist, wobei der Atlas, der Unterregionen gefährdeter Organe enthält, eine Wahrscheinlichkeitskarte der gefährdeten Unterregionen (P_{RR}) bereitstellt, wobei die Wahrscheinlichkeitskarte ein Datensatz ist, der für ein gegebenes Voxel eine Wahrscheinlichkeit aufweist, sich in einer Region zu befinden, die mit einer höheren Strahlenempfindlichkeit verbunden ist,
- eine zweite Vorrichtung, die Mittel zum Generieren (Ph2) mindestens einer für eine Einschränkung (C) repräsentativen Dateneinheit aufweist, zur Anwendung auf eine für den Patienten bestimmte Behandlung als Funktion der Wahrscheinlichkeitskarte der gefährdeten Unterregionen (PRR).

9. System zum Generieren von Daten nach Anspruch 8, **dadurch gekennzeichnet, dass** es Mittel zum Vorhersagen einer Toxizität für den Patienten als Funktion des mindestens einen für den zu behandelnden Patienten repräsentativen Bilds, und der mindestens einen für eine Einschränkung (C) repräsentativen, generierten Dateneinheit aufweist.

10. Computerprogrammprodukt, das von einem Kommunikationsnetzwerk heruntergeladen und/oder auf einem computerlesbaren Medium gespeichert und/oder von einem Mikroprozessor ausgeführt werden kann, **dadurch gekennzeichnet, dass** es Programmcodeanweisungen zum Ausführen eines Verfahrens zum Bereitstellen nach Anspruch 1 aufweist, wenn es auf einem Computer ausgeführt wird.

## Claims

1. Method for producing data representing radiotherapy treatment constraints associated with a patient to be treated, method **characterised in that** it comprises:
- a phase (Ph1) of mapping at least one current atlas (A₁) of a set of atlases (E_{(A)}) with at least one representative datum comprising at least one image representing a patient to be treated, said atlas containing sub-regions of organs at risk, providing a map of probability of sub-regions at risk (P_{RR}), said map of probability being a set of data comprising a probability that a given voxel is situated in a region associated with greater radio-sensitivity;
- a phase of generating (Ph2) at least one datum representing a constraint (C) to be applied to a treatment intended for said patient on the basis of said map of probability of sub-regions at risk (P_{RR}).

2. Method according to claim 1, **characterised in that** it further comprises a phase for planning at least one radiotherapy treatment on the basis of said at least one datum representing a constraint (C).

3. Method according to claim 1, **characterised in that** it further comprises a phase for predicting toxicity for said patient on the basis of said at least one image representing said patient to be treated and said at least one datum representing a constraint (C) generated.

4. Method according to claim 1, **characterised in that** said phase (Ph1) of mapping at least one current atlas (Aᵢ) of said set of atlases (E_{(A)}) comprises:
- a step for recalibrating, based on at least one defined recalibration parameter, at least one portion of said data from said current atlas on said at least one image representing said patient, providing a recalibrated current atlas containing raw probabilistic data;
- a step for calculating said map of probability of sub-regions at risk (P_{RR}), by means of said at least one image representing said patient, on the basis of said raw probabilistic data.

5. Method according to claim 4, **characterised in that** said phase (Ph1) of mapping at least one current atlas (Aᵢ) of said set of atlases (E_{(A)}) further comprises:
- a step for calculating a similarity between said current atlas and said at least one image representing said patient, providing a similarity value; and
- a step for weighting probabilistic data contained in said recalibrated atlas on the basis of said similarity value;
- and **in that** said step for calculating said map of probability of regions at risk (P_{RR}), by means of said at least one image representing said patient, is carried out on the basis of the weighted probabilistic data.

6. Method according to claim 1, **characterised in that** said phase (Ph2) of generating at least one datum representing a constraint (C) comprises:
- a step for thresholding (Ph20) the map of probability of sub-regions at risk (P_{RR}) on the basis of a predefined thresholding parameter (PS);
- a step for obtaining at least one 3D position of said region at risk for said patient, on the basis of said thresholded map of probability of sub-regions at risk.
- a step for generating said at least one datum representing a treatment constraint (C) on the basis of said position.

7. Device for producing data representing radiotherapy treatment constraints associated with a patient to be treated, device **characterised in that** it comprises:
- means for mapping at least one current atlas (Aᵢ) of a set of atlases (E_{(A)}) with at least one representative datum comprising at least one image representing a patient to be treated, said atlas containing sub-regions of organs at risk, providing a map of probability of sub-regions at risk (P_{RR}), said map of probability being a set of data comprising a probability that a given voxel is situated in a region associated with greater radio-sensitivity;
- means for generating (Ph2) at least one datum representing a constraint (C) to be applied to a treatment intended for said patient on the basis of said map of probability of sub-regions at risk (P_{RR}).

8. System for producing data representing radiotherapy treatment constraints associated with a patient to be treated, system **characterised in that** it comprises:
- a first device comprising
means for mapping at least one current atlas (Ai) of a set of atlases (E(A)) with at least one representative datum comprising at least one image representing a patient to be treated, said atlas containing sub-regions of organs at risk, providing a map of probability of sub-regions at risk (PRR), said map of probability being a set of data comprising a probability that a given voxel is situated in a region associated with greater radio-sensitivity;
- a second device comprising
means for generating (Ph2) at least one datum representing a constraint (C) to be applied to a treatment intended for said patient on the basis of said map of probability of sub-regions at risk (PRR).

9. System for producing data according to claim 8, **characterised in that** it comprises means for predicting toxicity for said patient on the basis of said at least one image representing said patient to be treated and said at least one datum representing a constraint (C) generated.

10. Computer software program which can be downloaded from a communication network and/or stored on a computer readable medium and/or executed by a microprocessor, **characterised in that** it comprises software instructions for executing a providing method according to claim 1 when it is executed on a computer.
